# EUROPEAN PATENT SPECIFICATION

(11) **EP 3 520 717 B1**
(45) Date of publication and mention of the grant of the patent: **05.05.2021**
(21) Application number: 17856363.1
(22) Date of filing: 28.09.2017
(51) Int. Cl.: A61B 17/128, A61B 17/10

(54) **CLIP REMOVAL DEVICE**
CLIPENTFERNUNGSVORRICHTUNG
DISPOSITIF DE RETRAIT DE CLIP

(30) Priority: 30.09.2016 JP 2016194646
(43) Date of publication of application: 07.08.2019
(73) Proprietor: Zeon Corporation, Tokyo 100-8246 (JP)
(72) Inventor: YAMADA, Tomohiro, Tokyo 100-8246 (JP)
(74) Representative: Maiwald Patent- und Rechtsanwaltsgesellschaft mbH
(86) International application number: PCT/JP2017/035303
(87) International publication number: WO 2018/062414

(56) References cited:
- EP-A1- 1 350 475
- WO-A1-2012/126477
- JP-A- 2007 125 264
- JP-A- 2009 189 705
- JP-A- 2013 532 535
- JP-A- 2015 192 724
- US-A1- 2007 191 883

## Description

### TECHNICAL FIELD

The present invention relates to a clip removal device that is used so as to remove a clip implanted in a living organism while gripping an internal body tissue by endoscopic manipulation.

### BACKGROUND ART

An endoscopic clip used for treatment such as endoscopic hemostasis, marking, and stitching is a medical instrument in which a pair of arm portions is moved toward each other by a fastening ring being moved (slid) and an internal body tissue is gripped at the front end of the arm portion or in the vicinity of the front end as disclosed in, for example, Patent Document 1.

The endoscopic clip is attached to the distal end of a clip device passed through an endoscopic treatment instrument guide tube and is guided to the internal body tissue to be treated. Then, with the internal body tissue (such as a mucous membrane) being gripped, the endoscopic clip is removed from the clip device and implanted into the body by manipulation of a control unit provided on the proximal end side of the clip device.

After a treatment object such as hemostasis is achieved, in some cases, the gripping of the internal body tissue by the clip implanted in the living organism may be released and the clip may be taken out of the body. Patent Document 2 and Patent Document 3 disclose clip removal devices as release devices to that end. According to these techniques, a fastening ring for arm-closing of a clip is slid to the base end side of the clip, and then the clip is arm-opened by its own elasticity and removed from the internal body tissue.

However, in the clip removal devices according to the related art, the very small fastening ring needs to be held and slid relatively to the clip. Besides, it is impossible to slide the fastening ring unless the posture of the distal end of the clip removal device is accurately set with respect to the posture of the clip. As a result, the clip removal devices according to the related art have problems in that a fastening ring movement manipulation is not easy and time-consuming work is entailed in some cases.

### CITATION LIST

### PATENT DOCUMENT

Patent Document 1: JP 2009-189705 A
Patent Document 2: JP 2007-125264 A
Patent Document 3: JP 2015-192724 A
Patent Document 4: EP a 350 475 A1
Patent Document 5: US 2007/191883 A1

EP 1 350 475 describes an end effector assembly for an endoscopic instrument that includes first and second end effectors, a screw and optionally a washer.

US 2007/191883 A1 describes an applying forceps for a clip, in particular, an applying forceps for an aneurysm clip, comprising two forceps legs which can be pivoted relative to each other and are designed to receive a clip.

### SUMMARY OF THE INVENTION

### PROBLEM TO BE SOLVED BY THE INVENTION

The invention has been made in view of the above circumstances, and an object of the invention is to provide a clip removal device configured so that a clip implanted in a body can be easily removed by endoscopic manipulation.

### MEANS FOR SOLVING THE PROBLEM

A clip removal device according to the invention is a clip removal device for endoscopically removing a clip having a pair of arm portions, a connecting portion interconnecting base ends of the pair of arm portions, and a fastening ring equipped so as to be movable along longitudinal directions of the pair of arm portions and implanted in the body in a state where the fastening ring is positioned on a front end side of the arm portion and a front end of the clip grips an internal body tissue. The clip removal device includes a sheath, a drive wire inserted through the sheath so as to be axially movable with respect to the sheath, and a removal device body provided on a distal end side of the drive wire. The removal device body is provided with a pair of longitudinal pieces which, when pulled into a distal end of the sheath, is closed so as to move toward each other and which, when caused to protrude from the distal end of the sheath, is opened so as to move away from each other. An engagement tapered section is formed in at least one of the longitudinal pieces such that a width of the longitudinal piece increases toward the front end side. In a process in which the pair of longitudinal pieces is pulled into the distal end of the sheath in a state where the arm portion of the clip is positioned between the pair of longitudinal pieces, the engagement tapered section engages with the fastening ring of the clip to move the fastening ring toward a base end of the clip.

In order to endoscopically remove the clip implanted in the body by using the clip removal device according to the invention, the distal end of the sheath is placed beside the clip gripping the internal body tissue first, and then the drive wire is pushed in with respect to the sheath by manipulation on the proximal end side of the clip removal device outside the body. Then, the removal device body is pushed out from the sheath and the front ends of the pair of longitudinal pieces are opened. Then, between the open longitudinal pieces, the removal device body and the sheath are moved to the position where the arm portion positioned closer to the internal body tissue side than the fastening ring of the clip is sandwiched.

Once the pair of longitudinal pieces is pulled into the distal end of the sheath in that state, the front end sides of the longitudinal pieces move toward each other. At the same time, the end of the fastening ring of the clip that is on the internal body tissue side (lower side/side close to the internal body tissue) engages with the engagement tapered section at a predetermined movement position along the longitudinal direction of the longitudinal piece. The engagement tapered section is formed such that the longitudinal piece increases in width toward the front end side. In the process in which the longitudinal piece is pulled into the distal end of the sheath, the engagement tapered section engages with the lower side of the fastening ring of the clip and moves the fastening ring toward the base end of the clip.

As a result, the fastening ring moves to the base end side along the arm portion of the clip, the front ends of the arm portions of the clip are mutually opened, and the clip is removed from the internal body tissue sandwiched between the front ends of the arm portions. As a result, it is possible to release the gripping of the internal body tissue by the clip.

Accordingly, in the clip removal device of the invention, the very small fastening ring does not have to be held and the pair of arm portions positioned on the internal body tissue side of the fastening ring of the clip has only to be sandwiched from the side of the clip gripping the internal body tissue unlike in the related art. Accordingly, the manipulation is easy and clip removal work can be performed in a short time. If direction adjustment is performed and a force that mutually closes the arm portions is applied when the arm portions of the clip are sandwiched by the longitudinal pieces of the removal device body, the fastening of the arm portion by the fastening ring is relaxed, and thus the frictional force already generated between the fastening ring and the arm portion is significantly relaxed. Accordingly, a movement of the fastening ring along the engagement tapered section is facilitated and the gripping of the internal body tissue is released with greater ease.

A raised facing surface raised inwards beyond a base end side facing surface of a base end of the longitudinal piece is formed on an inside facing surface of the longitudinal piece where the engagement tapered section is positioned. Although the raised facing surface may be formed on at least one of the pair of longitudinal pieces, it is more preferable that the raised facing surface is formed on each of both longitudinal pieces. The raised facing surfaces face each other, and thus it is easy to apply the force of mutual closing to the pair of arm portions of the clip sandwiched between the raised facing surfaces and the fastening by the fastening ring is loosened with ease. As a result, the fastening ring can be easily moved along the engagement tapered section. This action can be obtained by the raised facing surface being formed on one longitudinal piece as well.

A raised tapered surface may be formed in a boundary portion between the base end side facing surface and the raised facing surface. With this configuration, the longitudinal pieces are smoothly pulled into the distal end of the sheath in a state where the pair of arm portions is sandwiched between the pair of longitudinal pieces.

The raised tapered surface may be positioned at a boundary between the engagement tapered section and the base end of the longitudinal piece positioned closer to a base end side than the engagement tapered section or may be positioned closer to the base end side than the boundary along the longitudinal piece. Alternatively, the raised tapered surface may be positioned halfway on the inside facing surface of the longitudinal piece where the engagement tapered section is formed.

Preferably, a recessed facing surface recessed to a side opposite to the direction in which the raised facing surface is raised is formed on the front end side ahead of the raised facing surface along the longitudinal piece. The recessed facing surface may be formed in at least one of the pair of longitudinal pieces. More preferably, the recessed facing surface is formed in each of the longitudinal pieces. The recessed facing surfaces face each other, and thus the distance between the pair of arm portions of the clip sandwiched between the recessed facing surfaces increases. Accordingly, in a process in which the pair of longitudinal pieces is pulled into the distal end of the sheath, the fastening ring moved to the base end side of the arm portion by the engagement tapered section while a force is applied in the direction of mutual closing to the arm portions by the raised facing surfaces can be prevented from moving back to the front end side due to gravity as the distance between the arm portions increases owing to the presence of the recessed facing surfaces with the fastening ring moved to the base end side as a result of engagement with the longitudinal piece. Preventable as a result is a phenomenon in which the fastening ring moves to the front end side due to gravity and the clip is closed again when the clip is released from the longitudinal piece after releasing of internal body tissue gripping. This action can be obtained by the recessed facing surface being formed in one longitudinal piece as well.

The engagement tapered section may be formed on each of both width-direction sides at the front end of at least one of the longitudinal pieces. One of the engagement tapered sections positioned on both width-direction sides engages with the internal body tissue side end of the fastening ring so as to move the fastening ring for returning to the base end of the arm portion. The other moves along the surface of the internal body tissue and functions to lift the front end of the longitudinal piece with respect to the surface of the internal body tissue. As a result, it is possible to enhance the function of the engagement tapered section engaging with the internal body tissue side end of the fastening ring to move the fastening ring for returning to the base end of the arm portion. Further, when the engagement tapered sections are formed on both width-direction sides of the front end of the longitudinal piece, any engagement tapered section may be engaged with the fastening ring, and thus alignment between the engagement tapered section and the fastening ring is easy.

### BRIEF DESCRIPTION OF DRAWINGS

Fig. 1 is a plan view illustrating an overall configuration of a clip removal device according to an embodiment of the invention;
Fig. 2 is a cross-sectional view taken along line II-II of Fig. 1;
Fig. 3 is an enlarged perspective view of a main part of a removal device body of the clip removal device illustrated in Fig. 1;
Fig. 4 is an enlarged side view of a main part of the front end of a longitudinal piece of the removal device body illustrated in Fig. 3;
Fig. 5 is a perspective view of a main part illustrating the configuration of an endoscopic clip, which is an object to be removed by the clip removal device, in an arm-open state;
Fig. 6 is a perspective view of a main part illustrating the configuration of the endoscopic clip, which is an object to be removed by the clip removal device, in an arm-closed state;
Fig. 7 is a diagram illustrating an initial step for removing the clip illustrated in Fig. 6 by using the clip removal device illustrated in Figs. 1 to 4;
Fig. 8 is a diagram illustrating a clip removal step following Fig. 7;
Fig. 9 is a diagram illustrating a clip removal step following Fig. 8;
Fig. 10 is a diagram illustrating a clip removal step following Fig. 9;
Fig. 11 is a diagram illustrating a clip removal step following Fig. 10;
Fig. 12 is a perspective view of a removal device body illustrating a modification example of a clip removal device according to another embodiment of the invention; and
Fig. 13 is a perspective view of a removal device body illustrating a modification example of a clip removal device according to yet another embodiment of the invention.

### MODE(S) FOR CARRYING OUT THE INVENTION

Hereinafter, a clip removal device according to embodiments of the invention will be described in detail with reference to accompanying drawings.

### [Medical Clip]

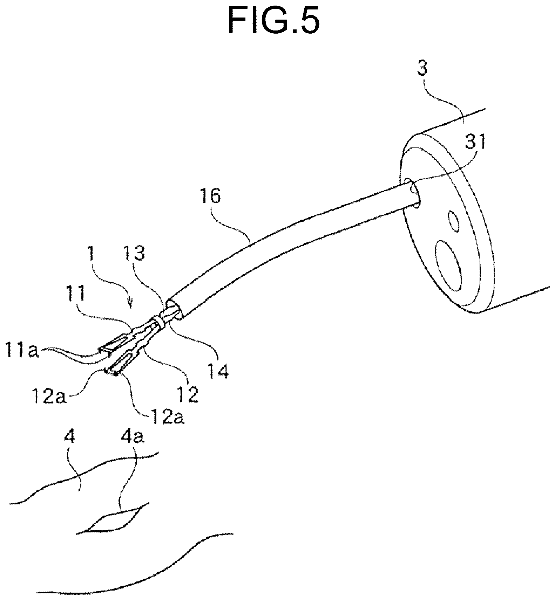

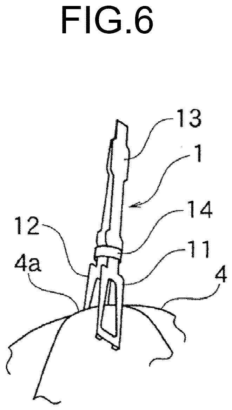

First, an endoscopic clip (medical clip) as an object to be removed by the clip removal device will be described with reference to Figs. 5 and 6. Although a clip having an arm plate portion formed in a plate shape as an arm portion will be described below, the endoscopic clip as an object to be removed by the clip removal device is not limited to the plate-shaped clip and may also be a clip that has an arm portion which has a rod shape (such as a columnar shape and a prismatic shape).

A clip 1 is configured to be provided with a pair of arm plate portions (arm portions) 11 and 12 and a connecting plate portion 13 bent in a substantially U shape. A fastening ring 14 opening and closing the pair of arm plate portions 11 and 12 is slidably and externally fitted to the connecting plate portion 13.

The pair of arm plate portions 11 and 12 is disposed such that the distance between the arm plate portions 11 and 12 increases toward the front ends in a state where no external force acts (such that the arm plate portions 11 and 12 are mutually opened in a substantially V shape toward the front ends (in an arm-opening manner)). One end (base end) of the base end of the arm plate portion 11 and one end (base end) of the base end of the arm plate portion 12 are integrally connected to one end of the connecting plate portion 13 and the other end of the connecting plate portion 13, respectively.

Claw portions 11a and 12a are integrally provided at the front ends of the arm plate portions 11 and 12, respectively. The claw portions 11a and 12a are formed by being bent inwards (that is, in the closing direction) at the front ends of the arm plate portions 11 and 12. Each of the claw portions 11a and 12a may have a recessed notch portion (not illustrated) at the intermediate part of the front end thereof.

The connecting plate portion 13, the pair of arm plate portions 11 and 12, and the pair of claw portions 11a and 12a constituting the endoscopic clip 1 can be formed by a single plate material being bent. Although not particularly limited, the plate thickness of the plate material constituting the endoscopic clip 1 is preferably 0.10 to 0.30 mm. An elastic metal plate is preferable as the plate material. For example, a stainless steel plate is used as the plate material.

A substantially cylindrical ring member constitutes the fastening ring 14. Alternatively, a spring formed by a wire rod being wound in a coil shape may constitute the fastening ring 14. The connecting plate portion 13 is inserted through a guide hole inside the fastening ring 14, and the fastening ring 14 is mounted (externally fitted) so as to be axially movable (slidable) between the outer periphery of the connecting plate portion 13 and the outer periphery of the arm plate portions 11 and 12. As illustrated in Fig. 6, in a case where the fastening ring 14 is slid from the position (release position) where the fastening ring 14 is externally fitted to the connecting plate portion 13 to the position (holding position) where the fastening ring 14 is externally fitted to the arm plate portions 11 and 12, the base ends of the pair of arm plate portions 11 and 12 are pulled into the inside (guide hole) of the fastening ring 14 and the arm plate portions 11 and 12 are arm-closed. In other words, the arm plate portions 11 and 12 are moved toward each other.

As illustrated in Fig. 5, in a state where the fastening ring 14 is disposed close to the base end side of the clip 1 (in the connecting plate portion 13), the arm plate portions 11 and 12 are open (arm-open) owing to the elasticity of the arm plate portions 11 and 12. As illustrated in Fig. 6, if necessary, the arm plate portions 11 and 12 can be gradually closed by the fastening ring 14 being moved (slid) to a position close to the front end side of the clip 1. Then, it is possible to maintain the arm plate portions 11 and 12 in a closed (arm-closed) state by moving the fastening ring 14 to the base ends of the arm plate portions 11 and 12.

The endoscopic clip 1 is attached to the distal end of a clip device 16 that is passed through a treatment instrument guide tube 31 of an endoscope 3 in a state where the fastening ring 14 is disposed in the connecting plate portion 13 and the arm plate portions 11 and 12 are open. Then, the endoscopic clip 1 is guided to an internal body tissue 4 through the treatment instrument guide tube 31 of the endoscope 3 for treatment of, for example, a place having a defective part formed as a result of lesion site excision. Subsequently, the fastening ring 14 is slid to the base ends of the arm plate portions 11 and 12 and the arm plate portions 11 and 12 are closed by manipulation of a control unit provided at the proximal end of the clip device 16. As a result, the front ends of the arm plate portions 11 and 12 sandwich and grip a defective part 4a of the internal body tissue (such as a mucous membrane) 4 formed as a result of lesion site excision as illustrated in Fig. 6. The defective part 4a is stitched as a result, and the endoscopic clip 1 is removed from the clip device 16 and implanted into the body in that state.

The clip removal device (clip remover) is used in a case where, for example, the clip 1 needs to be removed after implanted into the body as described above.

### [Clip Removal Device]

### First Embodiment

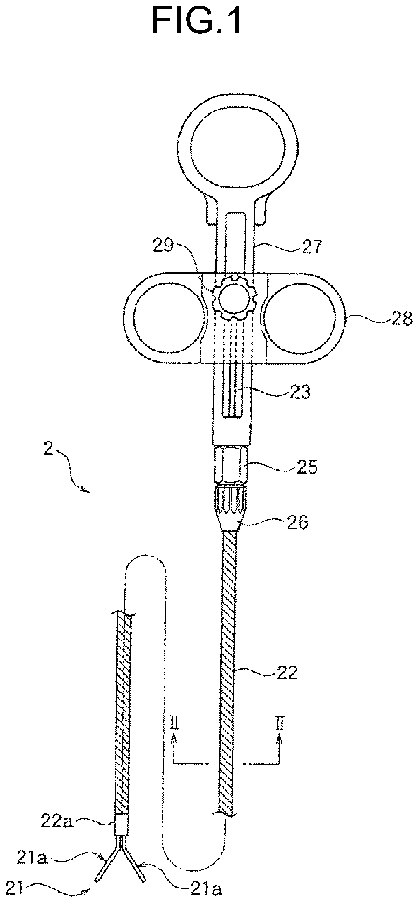

Hereinafter, the clip removal device according to a first embodiment of the invention will be described with reference to Figs. 1 to 11. A clip removal device 2 has a sheath 22, a drive wire 23 inserted through the sheath 22 so as to be axially movable with respect to the sheath 22, and a removal device body 21 provided on the distal end side of the drive wire 23. A base portion 27 is connected to the proximal end of the sheath 22 by a sheath side lock member 26 and a base side lock member 25.

A slider portion 28 is slidably attached to the base portion 27, and the proximal end of the drive wire 23 reaches the slider portion 28 through respective through holes of the sheath side lock member 26 and the base side lock member 25 and is releasably fixed to the slider portion 28 via a lock screw 29.

By the slider portion 28 being slid to the distal end side with respect to the base portion 27, the drive wire 23 moves to the distal end side with respect to the sheath 22 and the removal device body 21 provided on the distal end side of the drive wire 23 is pushed out from a distal end 22a of the sheath 22. As a result, a pair of longitudinal pieces 21a of the removal device body 21 is opened. In contrast, by the slider portion 28 being slid to the proximal end side with respect to the base portion 27, the removal device body 21 provided on the distal end side of the drive wire 23 is pulled into the distal end 22a of the sheath 22, and then the pair of longitudinal pieces 21a is closed.

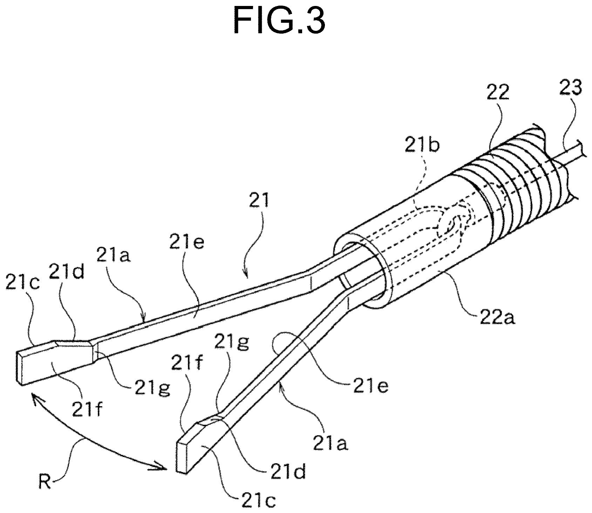

As illustrated in Fig. 3, the distal end of the drive wire 23 is connected to a base folded piece 21b of the removal device body 21. By the slider portion 28 illustrated in Fig. 1 being advanced and retracted with respect to the base portion 27, the drive wire 23 axially moves inside the sheath 22. As a result, the pair of longitudinal pieces 21a of the removal device body 21 is capable of protruding from or being pulled into the distal end 22a of the sheath 22 illustrated in Fig. 3. Although the drive wire 23 and the removal device body 21 are connected to each other by a ring-shaped member being connected to the distal end of the drive wire 23 and the removal device body 21 being inserted through the ring as illustrated in Fig. 3 in the present embodiment, methods for the connection are not particularly limited. For example, simple and direct connection based on joining means such as welding may be adopted.

The sheath 22 is a tubular member. The sheath 22 is desirably flexible. Although a simple tube made of resin or the like may be used as the sheath 22, a coil tube is used as the sheath 22 in the present embodiment. Usable as the coil tube is a flat coil tube resulting from spiral winding of a long flat plate made of metal (such as stainless steel) or the like. Further, in the present embodiment, the distal end 22a of the sheath 22 is configured by a metal (such as stainless steel) pipe substantially equal in inner diameter and outer diameter to the coil tube being connected to the distal end side of the coil tube constituting the sheath 22. By the metal pipe constituting the distal end 22a of the sheath 22, the longitudinal pieces 21a can be more reliably closed against the force that opens the longitudinal pieces 21a by elasticity in directions away from each other when the pair of longitudinal pieces 21a is pulled into the distal end 22a. The distal end 22a of the sheath 22 does not necessarily have to be configured by means of a metal pipe. For example, a coil tube may constitute the entire sheath 22.

Alternatively, a round wire coil tube or a coil tube with a flat inner surface may be used as the sheath 22. Alternatively, a wire tube may be used as the sheath 22. The wire tube is a tube made of, for example, a hollow stranded wire formed by a plurality of wires (cables) made of metal (such as stainless steel) or the like being spirally twisted so as to be hollow.

The drive wire 23 is made of a flexible wire. In the present embodiment, a wire rope is used as the drive wire 23. The wire rope is a rope made of, for example, a stranded wire formed by a plurality of wires (cables) made of metal (such as stainless steel) or the like being spirally twisted. Alternatively, another wire may be used as the drive wire 23 and a wire tube and so on may be used as the drive wire 23, for example. Alternatively, a single wire rod may constitute the drive wire 23.

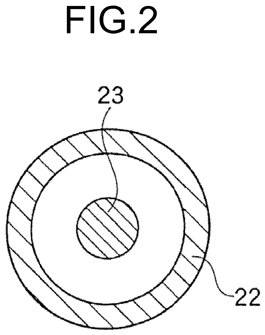

As illustrated in Figs. 1 and 2, the drive wire 23 is inserted through the sheath 22 so as to be movable along the axial direction with respect to the sheath 22. As illustrated in Fig. 3, the removal device body 21 in which the base folded piece 21b is connected to the front end (distal end) of the drive wire 23 integrally has the pair of longitudinal pieces 21a. A front end piece 21c as a part of the longitudinal piece 21a is integrally formed at the front end of the longitudinal piece 21a. The pair of longitudinal pieces 21a integrated by the base folded piece 21b can be formed by bending of an elongated elastic plate piece such as a stainless steel plate, for example. Alternatively, the pair of longitudinal pieces 21a may be molded by means of bending of an elongated elastic rod material such as a stainless steel rod, for example. Alternatively, the pair of longitudinal pieces 21a integrated by the base folded piece 21b may be made of an elastic synthetic resin or the like by injection molding.

The pair of longitudinal pieces 21a has a shape in which the distance between the pair of longitudinal pieces 21a increases from the base toward the front ends. In a state where no external force acts, the distance between the front ends of the pair of longitudinal pieces 21a along an opening and closing direction R exceeds the inner diameter of the distal end 22a of the sheath 22. In a state where the longitudinal pieces 21a are pulled into the distal end 22a of the sheath 22, the front ends of the longitudinal pieces 21a are closed so as to move toward each other. In addition, as illustrated in Fig. 3, the front ends of the longitudinal pieces 21a are opened so as to move away from each other by the elasticity of the longitudinal pieces 21a and the base folded piece 21b in a state where the front ends of the longitudinal pieces 21a protrude from the distal end 22a of the sheath 22.

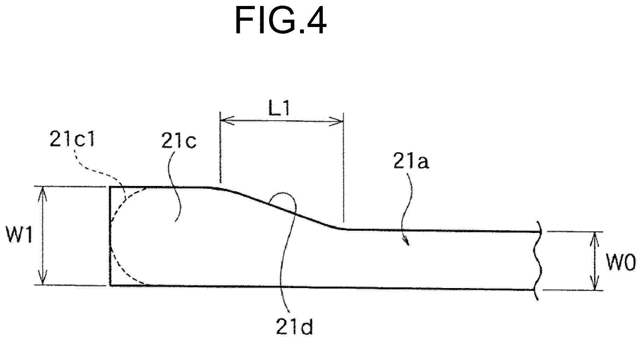

As illustrated in Fig. 4, a width W1 of the front end piece 21c at the front end of each longitudinal piece 21a (width in a direction substantially perpendicular to the opening and closing direction R illustrated in Fig. 3) preferably exceeds a width W0 of the base end of the longitudinal piece 21a. Preferably, W1/W0 is 1.5 to 2.0. The width W1 of the front end piece 21c may exceed or be exceeded by the inner diameter of the distal end 22a of the sheath 22. In a case where the inner diameter of the distal end 22a of the sheath 22 exceeds the width W1 of the front end piece 21c, not only the base end of the longitudinal piece 21a but also the front end piece 21c can be pulled into the distal end 22a of the sheath 22.

In the present embodiment, engagement tapered sections 21d are formed on the width-direction upper edges of the longitudinal pieces 21a for a width gradually increasing from the respective base ends of the longitudinal pieces 21a toward the front end pieces 21c. In the present embodiment, the width-direction lower edge of each longitudinal piece 21a is formed in a substantially linear shape with the front end piece 21c included and is not provided with the engagement tapered section 21d. Incidentally, in the present embodiment, a wide part with a constant width is formed at the front end of the engagement tapered section 21d in the front end piece 21c. Alternatively, only the engagement tapered section 21d may be formed or a linear inclined portion or a curved inclined portion 21c1 (see Fig. 4) decreasing in width toward the front end may be provided at the front end of the engagement tapered section 21d.

A longitudinal length L1 of the engagement tapered section 21d illustrated in Fig. 4 is determined from, for example, the relationship between the length L1 and the width W0 of the base end of the longitudinal piece 21a. Preferably, L1/W0 is 1 to 2. In the present embodiment, the front end piece 21c on which the engagement tapered section 21d is formed is provided with a raised facing surface 21f raised inwards beyond a base end side facing surface 21e at the base end of the longitudinal piece 21a.

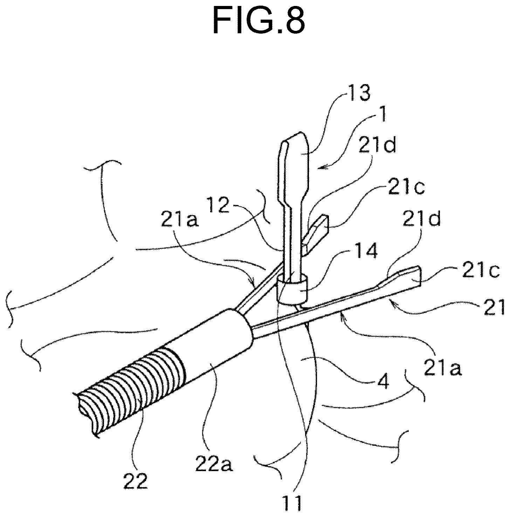

Incidentally, the base end of the longitudinal piece 21a is the part of the longitudinal piece 21a that is positioned closer to the base end side than the engagement tapered section 21d and may include the base folded piece 21b. The width of the base folded piece 21b and the width W0 of the base end of the longitudinal piece 21a may differ from each other although the widths are substantially equal to each other in the present embodiment. Further, the width W0 of the base end of the longitudinal piece 21a may be somewhat different along the longitudinal direction. As illustrated in Fig. 8, for example, at the base ends of the longitudinal pieces 21a and 21a sandwiched between the fastening ring 14 of the clip 1 and the surface of the internal body tissue 4, the width W0 may be small within a strength-ensuring range such that sandwiching at that part is facilitated.

In addition, the facing surfaces 21e and 21f are the surfaces of the pair of mutual longitudinal pieces 21a that face each other. Although the facing surfaces 21e and 21f are flat surfaces in the present embodiment, the facing surfaces 21e and 21f do not necessarily have to be flat and each may be a convex curved surface instead. However, preferably, the facing surfaces 21f are raised inwards (in the direction of facing) higher than the facing surfaces 21e.

Preferably, a raised tapered surface 21g is formed at the boundary between the facing surface 21e and the facing surface 21f, and the raised tapered surface 21g is raised gradually inwards toward the front end side. In the present embodiment, the raised tapered surface 21g is formed in the vicinity of the boundary between the base end of the longitudinal piece 21a and the engagement tapered section 21d of the front end piece 21c. Alternatively, the raised tapered surface 21g may be formed closer to the base end side than the boundary along the longitudinal piece 21a. Alternatively, the raised tapered surface 21g may be formed halfway on the facing surface of the longitudinal piece on which the engagement tapered section 21d is formed. Preferably, the raised tapered surface 21g is formed in the vicinity of the boundary between the base end of the longitudinal piece 21a and the engagement tapered section 21d of the front end piece 21c or slightly closer to the base end side than the boundary along the longitudinal piece 21a.

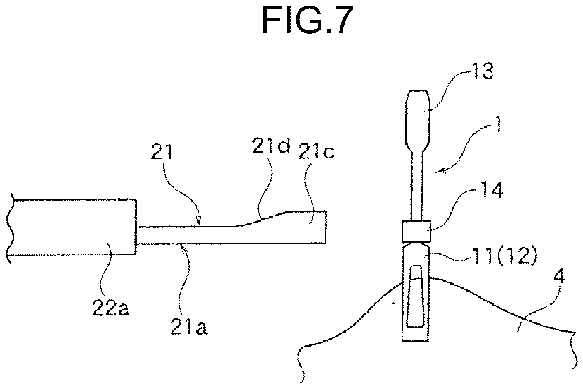

Next, a method for using the above-described clip removal device 2 will be described with reference to Figs. 7 to 11. Incidentally, in the clip 1 that is illustrated in Figs. 7 to 11, the fastening ring 14 is set at the holding position, at which the fastening ring 14 is positioned at base ends 11b and 12b of the pair of arm plate portions 11 and 12, after sliding from the release position, at which the fastening ring 14 is positioned in the connecting plate portion 13. In that state, the clip 1 is implanted into the body in a state where the internal body tissue 4 (such as a mucous membrane) is gripped by the claw portions of the pair of arm plate portions 11 and 12.

First, the sheath 22 of the clip removal device 2 illustrated in Fig. 1 is inserted instead of the clip device 16 via, for example, the treatment instrument guide tube 31 of the endoscope 3 illustrated in Fig. 5. Then, as illustrated in Fig. 7, the distal end 22a of the sheath 22 is positioned near the clip 1 that is implanted in the body and to be released from gripping. In other words, the distal end 22a of the sheath 22 is brought closer to the side of the clip 1 gripping the internal body tissue 4. Before and after this manipulation, the slider portion 28 is slid to the distal end side with respect to the base portion 27 of the clip removal device 2 illustrated in Fig. 1, the removal device body 21 provided on the distal end side of the drive wire 23 is caused to protrude from the distal end 22a of the sheath 22, and the pair of longitudinal pieces 21a is opened.

Before and after that, the base portion 27 and the slider portion 28 are integrally rotated for positional relationship adjustment between the clip 1 and the engagement tapered section 21d of the longitudinal piece 21a. In other words, assuming that the direction of the connecting plate portion 13 of the clip 1 is upward with respect to the internal body tissue 4, the longitudinal piece 21a is rotated about the axis of the sheath 22 such that the engagement tapered section 21d of the longitudinal piece 21a faces upwards.

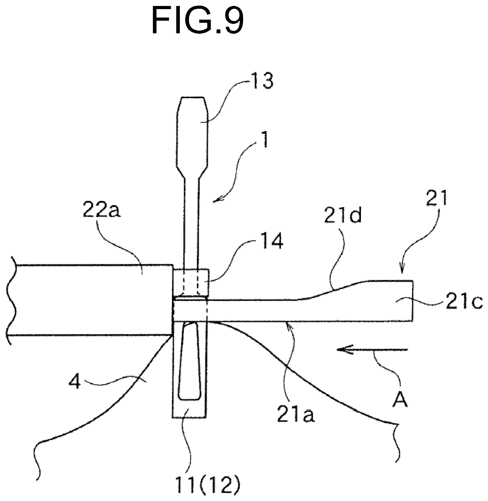

Subsequently, as illustrated in Figs. 8 and 9, the removal device body 21 and the distal end 22a of the sheath 22 are moved, between the open longitudinal pieces 21a and 21a, to the position where the force is applied that closes the arm plate portions 11 and 12 by sandwiching the arm plate portions 11 and 12 positioned on the internal body tissue 4 side (lower side) of the fastening ring 14 of the clip 1.

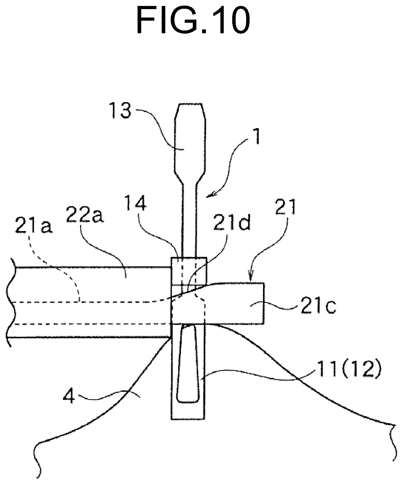

As illustrated in Figs. 9 and 10, in that state, the pair of longitudinal pieces 21a and 21a is pulled in a longitudinal direction A into the distal end 22a of the sheath 22. Then, the front end pieces 21c of the longitudinal pieces 21a and 21a move toward each other (are closed). As a result, the frictional force between the longitudinal pieces 21a and 21a and the fastening ring 14 that fastened the longitudinal pieces 21a and 21a is significantly relaxed. Then, at the same time, the lower side of the fastening ring 14 of the clip 1 engages with the engagement tapered sections 21d at predetermined movement positions of the longitudinal pieces 21a and 21a along the longitudinal direction A. The engagement tapered section 21d is formed so as to increase in width toward the front end side. In a process of pulling into the distal end 22a of the sheath 22, the engagement tapered section 21d engages with the lower side of the fastening ring 14 of the clip 1 and moves the fastening ring 14 toward the base end of the clip 1 (upwards/in a direction away from the internal body tissue 4).

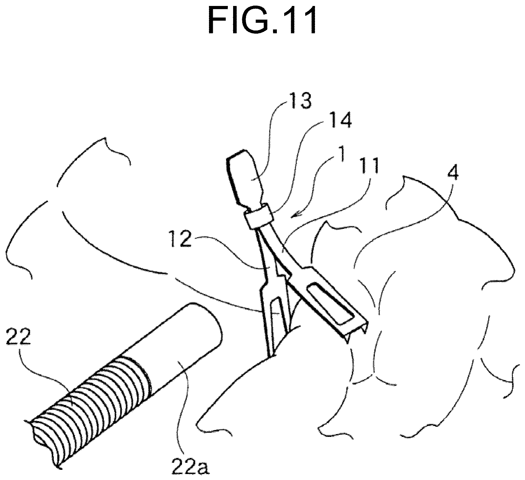

As a result, the fastening ring 14 moves to the base end side along the arm plate portions 11 and 12 of the clip 1, the front ends of the arm plate portions 11 and 12 of the clip 1 are mutually opened, and the clip 1 is removed from the internal body tissue 4 sandwiched between the front ends of the arm plate portions 11 and 12 as illustrated in Fig. 11. As a result, it is possible to release the gripping of the internal body tissue 4 by the clip 1. Incidentally, the gripping-released clip 1 is separately taken out of the body.

Accordingly, in the clip removal device 2 of the present embodiment illustrated in Fig. 1, the very small fastening ring 14 does not have to be held and the arm plate portions 11 and 12 positioned on the lower side of the fastening ring 14 of the clip 1 have only to be sandwiched from the side with respect to the clip 1 gripping the internal body tissue 4 unlike in the related art. Accordingly, the manipulation is easy and removal work for the clip 1 can be performed in a short time. Incidentally, in the present embodiment, the arm plate portions 11 and 12 are sandwiched with the longitudinal pieces 21a and 21a such that the closing force is applied to the arm plate portions 11 and 12 of the clip 1. In a case where it is possible to move the fastening ring 14 toward the base end of the clip 1 simply by the action of the engagement tapered section 21d even without loosening the fastening force of the fastening ring 14, however, the respective side surfaces of the arm plate portions 11 and 12 may be sandwiched so as to abut against the longitudinal pieces 21a and 21a.

Further, as illustrated in Fig. 3, in the present embodiment, the facing surfaces of the longitudinal pieces 21a and 21a where the engagement tapered sections 21d are positioned are provided with the raised facing surfaces 21f raised inwards beyond the base end side facing surfaces 21e at the base ends of the longitudinal pieces 21a and 21a. Although the raised facing surface 21f may be formed on at least one of the pair of longitudinal pieces 21a and 21a, the raised facing surface 21f is formed on each of the longitudinal pieces 21a and 21a in the present embodiment.

In the present embodiment, the raised facing surfaces 21f and 21f face each other, and thus it is easy to apply the force of mutual closing to the pair of arm plate portions 11 and 12 of the clip 1 sandwiched between the raised facing surfaces 21f and 21f and the fastening by the fastening ring 14 is loosened with ease. As a result, the fastening ring 14 can be easily moved along the engagement tapered section 21d as illustrated in Fig. 10. Incidentally, this action can be obtained by the raised facing surface 21f illustrated in Fig. 3 being formed on one longitudinal piece 21a as well.

In addition, as illustrated in Fig. 3, in the present embodiment, the raised tapered surface 21g is formed in the boundary portion between the base end side facing surface 21e and the raised facing surface 21f. With this configuration, the longitudinal pieces 21a and 21a are smoothly pulled into the distal end 22a of the sheath 22 in a state where the pair of arm plate portions 11 and 12 is sandwiched between the pair of longitudinal pieces 21a and 21a as illustrated in Figs. 9 and 10.

### Second Embodiment

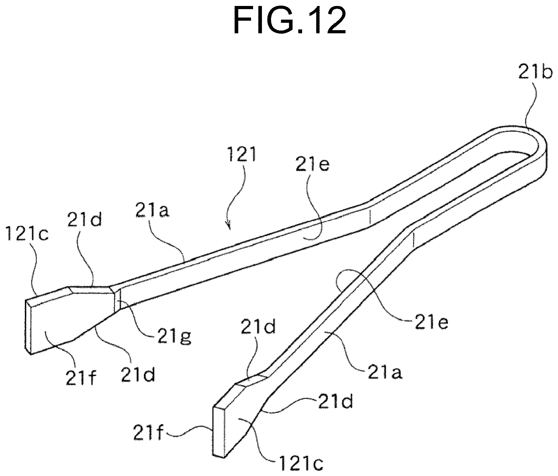

Next, a second embodiment of the invention will be described with reference to Fig. 12. In the present embodiment, the configuration of a removal device body 121 is changed with respect to the first embodiment described above. It is to be noted that description of parts common to the first and second embodiments is omitted below and that the common parts are denoted by the same reference numerals in the drawing.

In the present embodiment, front end pieces 121c and 121c as a part of the respective longitudinal pieces 21a and 21a are formed on the front end sides of the pair of longitudinal pieces 21a and 21a of the removal device body 121 and the engagement tapered sections 21d are formed on both width-direction sides of each of the front end pieces 121c and 121c. In the engagement tapered sections 21d positioned on both width-direction sides, the upper engagement tapered sections 21d engage with the fastening ring 14 so as to engage with the lower side of the fastening ring 14 illustrated in Fig. 10 and move the fastening ring 14 to the base end sides (upper sides) of the arm plate portions 11 and 12.

In addition, the lower engagement tapered sections 21d illustrated in Fig. 12 move along the surface of the internal body tissue 4 illustrated in Fig. 10 and function to lift the front end pieces 121c of the longitudinal pieces 21a with respect to the surface of the internal body tissue 4. As a result, it is possible to enhance the function of engaging with the lower side of the fastening ring 14 to move the fastening ring 14 to the base end sides (upper sides) of the arm plate portions 11 and 12. In addition, when the engagement tapered sections 21d are formed on both width-direction sides of the front end pieces 121c of the longitudinal pieces 21a, any engagement tapered section 21d may be engaged with the fastening ring 14, and thus the upper and lower sides of the removal device body 121 do not have to be considered and alignment between the engagement tapered section 21d and the fastening ring 14 is easy.

Incidentally, in the present embodiment, the pair of engagement tapered sections 21d is formed in the width direction in each of the pair of longitudinal pieces 21a and 21a. Alternatively, the pair of engagement tapered sections 21d may be formed in the width direction in only one of the longitudinal pieces 21a.

### Third Embodiment

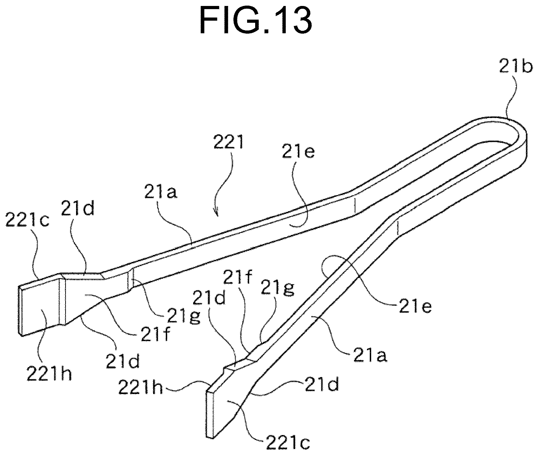

Next, a third embodiment of the invention will be described with reference to Fig. 13. In the present embodiment, the configuration of a removal device body 221 is changed with respect to the second embodiment described above. It is to be noted that description of parts common to the second and third embodiments is omitted below and that the common parts are denoted by the same reference numerals in the drawing.

In the present embodiment, a front end piece 221c is formed on the front end side of the longitudinal piece 21a of the removal device body 221, recessed facing surfaces 221h are formed on the front end sides of the front end pieces 221c that are ahead of the raised facing surfaces 21f along the longitudinal pieces 21a and 21a, and the recessed facing surface 221h is recessed to the side that is opposite to the direction in which the raised facing surface 21f is raised. The recessed facing surface 221h may be formed in at least one of the longitudinal pieces 21a. More preferably, the recessed facing surface 221h is formed in each of the longitudinal pieces 21a.

The recessed facing surfaces 221h face each other, and thus the distance between the pair of arm plate portions 11 and 12 of the clip 1 in Fig. 10 sandwiched between the recessed facing surfaces 221h increases. Accordingly, in a process during pulling of the longitudinal piece 21a into the distal end 22a of the sheath 22, the fastening ring 14 is moved to the base ends (upper sides) of the arm plate portions 11 and 12 by the engagement tapered sections 21d while a force is applied in the direction of mutual closing of the arm plate portions 11 and 12 by the raised facing surfaces 21f, and then the distance between the arm plate portions 11 and 12 is increased by the recessed facing surfaces 221h with the fastening ring 14 remaining supported by the edges of the front end pieces 221. Once the support of the fastening ring 14 is released before the distance between the arm plate portions 11 and 12 is increased, the fastening ring 14 moves again to the front end side due to gravity, and then the clip 1 may be closed unintendedly. However, this phenomenon can be prevented with the present embodiment. Incidentally, this action can be obtained by the recessed facing surface 221h illustrated in Fig. 13 being formed in the front end piece 221c of at least one longitudinal piece 21a as well.

In addition, in the third embodiment illustrated in Fig. 13, the raised tapered surface 21g is positioned at the boundary between the base end of the longitudinal piece 21a and the engagement tapered section 21d. In the present embodiment, however, the raised tapered surface 21g is positioned closer to the base end side than the boundary along the longitudinal piece 21a. In this case and in a process in which the longitudinal piece 21a is pulled into the distal end 22a of the sheath 22 (process from Fig. 9 to Fig. 10), the arm plate portions 11 and 12 of the clip 1 are tightly sandwiched between the raised facing surfaces 21f, and the lower end of the fastening ring 14 subsequently engages with the engagement tapered section 21d, and the fastening ring 14 is moved upwards along the arm plate portions 11 and 12 as a result. Incidentally, the raised tapered surface 21g illustrated in Fig. 13 may be positioned halfway on the facing surface of the longitudinal piece 21a where the engagement tapered section 21d is positioned.

### EXPLANATIONS OF LETTERS OR NUMERALS

- 1: ENDOSCOPIC CLIP
11, 12 ARM PLATE PORTION (ARM PORTION)
13 CONNECTING PLATE PORTION
14 FASTENING RING
16 CLIP DEVICE
- 2 CLIP: REMOVAL DEVICE
21, 121, 221 REMOVAL DEVICE BODY
21a LONGITUDINAL PIECE
21b BASE FOLDED PIECE
21c, 121c, 221c FRONT END PIECE
21d ENGAGEMENT TAPERED SECTION
21e BASE END SIDE FACING SURFACE
21f RAISED FACING SURFACE
21g RAISED TAPERED SURFACE
221h RECESSED FACING SURFACE
22 SHEATH
22a DISTAL END
23 DRIVE WIRE
25 BASE SIDE LOCK MEMBER
26 SHEATH SIDE LOCK MEMBER
27 BASE PORTION
28 SLIDER PORTION
29 LOCK SCREW
- 3: ENDOSCOPE
- 4: INTERNAL BODY TISSUE
4a DEFECTIVE PART

## Claims

1. A clip removal device (2) for endoscopically removing a clip (1) having a pair of arm portions (11, 12), a connecting portion (13) interconnecting base ends of the pair of arm portions (11, 12), and a fastening ring (14) equipped so as to be movable along longitudinal directions of the pair of arm portions (11, 12) and implanted in the body in a state where the fastening ring (14) is positioned on a front end side of the arm portions (11, 12) and where a front end of the clip grips an internal body tissue (4),
the clip removal device (2) comprising:
a sheath (22);
a drive wire (23) inserted through the sheath (22) so as to be axially movable with respect to the sheath (22); and
a removal device body (21, 121, 221) provided on a distal end side of the drive wire (23), wherein
the removal device body (21, 121, 221) is provided with a pair of longitudinal pieces (21a) which, when pulled into a distal end (22a) of the sheath (22), is closed so as to move toward each other and which, when caused to protrude from the distal end (22a) of the sheath (22), is opened so as to move away from each other,
an engagement tapered section (21d) is formed in at least one of the longitudinal pieces such that a width of the longitudinal piece (21a) increases toward the front end side,
a raised facing surface (21f) raised inwards beyond a base end side facing surface (21e) of a base end of the longitudinal piece (21a) is formed on an inside facing surface of the longitudinal piece (21a) where the engagement tapered section (21d) is positioned, and
in a process in which the pair of longitudinal pieces (21a) is pulled into the distal end (22a) of the sheath (22) in a state where the arm portions (11, 12) of the clip (1) are positioned between the pair of longitudinal pieces (21a), the engagement tapered section (21d) engages with the fastening ring (14) of the clip (1) to move the fastening ring (14) toward a base end of the clip (1).

2. The clip removal device according to claim 1, wherein a raised tapered surface (21g) is formed in a boundary portion between the base end side facing surface (21e) ES:YW and the raised facing surface (21f).

3. The clip removal device according to claim 2, wherein the raised tapered surface (21g) is positioned at a boundary between the engagement tapered section (21d) and the base end of the longitudinal piece (21a) or positioned closer to a base end side than the boundary along the longitudinal piece (21a), the base end of the longitudinal piece (21a) being the part of the longitudinal piece (21a) being positioned closer to the base end side than the engagement tapered section (21d).

4. The clip removal device according to any one of claims 1 to 3, wherein a recessed facing surface (221h) recessed to a side opposite to the direction in which the raised facing surface (21f) is raised is formed on the front end side ahead of the raised facing surface (21f) along the longitudinal piece (21a).

5. The clip removal device according to any one of claims 1 to 4, wherein the engagement tapered section (21d) is formed on each of both width-direction sides at the front end of at least one of the longitudinal pieces (21a).

## Patentansprüche

1. Eine Clipentfernungsvorrichtung (2) zum endoskopischen Entfernen eines Clips (1) umfassend ein Paar von Armabschnitten (11, 12), einen Verbindungsabschnitt (13), der die Basisenden des Paares von Armabschnitten (11, 12) miteinander verbindet, und einen Befestigungsring (14), der derart ausgebildet ist, um entlang der Längsrichtungen des Paares von Armabschnitten (11, 12) bewegbar zu sein, und in dem Körper in einem Zustand implantiert ist, in welchem der Befestigungsring (14) an einer vorderen Endseite des Armabschnitts (11, 12) positioniert ist und in welchem ein vorderes Ende des Clips ein inneres Körpergewebe (4) greift, die Clipentfernungsvorrichtung (2) umfassend:
eine Hülle (22);
einen Antriebsdraht (23), der durch die Hülle (22) eingeführt wird, um in Bezug auf die Hülle (22) axial bewegbar zu sein; und
einen Entfernungsvorrichtungskörper (21, 121, 221), der an einer distalen Endseite des Antriebsdrahtes (23) vorgesehen ist, wobei
der Entfernungsvorrichtungskörper (21, 121, 221) mit einem Paar von Längsstücken (21a) versehen ist, welches beim Ziehen in ein distales Ende (22a) der Hülle (22) geschlossen ist, um sich aufeinander zu bewegen, und welches bei dem Führen zum Herausragen aus dem distalen Ende (22a) der Hülle (22) geöffnet ist, um sich voneinander zu entfernen,
ein sich verjüngender Eingriffsabschnitt (21d) in mindestens einem der Längsstücke ausgestaltet ist, so dass eine Breite des Längsstücks (21a) zur vorderen Endseite hin zunimmt,
eine erhabene Stirnfläche (21f), die nach innen über eine Basisendseite zugewandte Fläche (21e) eines Basisendes des Längsstücks (21a) hinaus angehoben ist, an einer nach innen zugewandten Fläche des Längsstücks (21a) ausgestaltet ist, an der der sich verjüngende Eingriffsabschnitt (21d) positioniert ist, und
in einem Prozess, in welchem das Paar von Längsstücken (21a) in das distale Ende (22a) der Hülle (22) in einem Zustand gezogen wird, in dem die Armabschnitte (11, 12) des Clips (1) zwischen dem Paar von Längsstücken (21a) positioniert sind, der sich verjüngende Eingriffsabschnitt (21d) in den Befestigungsring (14) des Clips (1) eingreift, um den Befestigungsring (14) in Richtung eines Basisendes des Clips (1) zu bewegen.

2. Die Clipentfernungsvorrichtung nach Anspruch 1, wobei eine erhabene, sich verjüngende Fläche (21g) in einem Grenzabschnitt zwischen der Basisendseite zugewandten Fläche (21e) und der erhabenen Stirnfläche (21f) ausgestaltet ist.

3. Die Clipentfernungsvorrichtung nach Anspruch 2, wobei die erhabene, sich verjüngende Fläche (21g) an einer Grenze zwischen dem sich verjüngenden Eingriffsabschnitt (21d) und dem Basisende des Längsstücks (21a) positioniert ist oder näher an einer Basisendseite als der Grenze entlang der Längsstücke (21a) positioniert ist, wobei das Basisende der Längsstücke (21a) der Teil des Längsstücke (21a), die näher an der Basisendseite als dem sich verjüngenden Eingriffsabschnitt (21d) positioniert ist.

4. Die Clipentfernungsvorrichtung nach einem der Ansprüche 1 bis 3, wobei eine vertiefte Stirnfläche (221h), die zu einer Seite vertieft ist, die der Richtung entgegengesetzt ist, in welcher die erhabene Stirnfläche (21g) angehoben ist, an der vorderen Endseite vor der erhabenen Stirnfläche (21f) entlang der Längsstück (21a) ausgestaltet ist.

5. Die Clipentfernungsvorrichtung nach einem der Ansprüche 1 bis 4, wobei der sich verjüngende Eingriffsabschnitt (21d) an jeder der beiden Seiten in Breitenrichtung an dem vorderen Ende der mindestens einem der Längsstücken (21a) ausgebildet ist.

## Revendications

1. Dispositif de retrait d'agrafe (2) pour retirer de façon endoscopique une agrafe (1) comportant une paire de parties de bras (11, 12), une partie de liaison (13) interconnectant des extrémités de base de la paire de parties de bras (11, 12), et une bague de fixation (14) agencée de façon à être mobile le long de directions longitudinales de la paire de parties de bras (11, 12) et implantée dans le corps dans un état dans lequel la bague de fixation (14) est positionnée sur un côté d'extrémité avant des parties de bras (11, 12), et dans lequel une extrémité avant de l'agrafe saisit un tissu corporel interne (4),
le dispositif de retrait d'agrafe (2) comprenant :
un manchon (22) ;
un fil d'entraînement (23) inséré à travers le manchon (22) de façon à être axialement mobile par rapport au manchon (22) ; et
un corps de dispositif de retrait (21, 121, 221) disposé sur un côté d'extrémité distale du fil d'entraînement (23), dans lequel :
le corps de dispositif de retrait (21, 121, 221) est muni d'une paire de pièces longitudinales (21a) qui, lorsqu'elles sont tirées dans une extrémité distale (22a) du manchon (22), sont fermées de façon à se rapprocher l'une de l'autre, et qui, lorsqu'elles sont amenées à faire saillie à partir de l'extrémité distale (22a) du manchon (22), sont ouvertes de façon à s'éloigner l'une de l'autre,
une section effilée de prise (21d) est formée dans au moins l'une des pièces longitudinales de telle sorte qu'une largeur de la pièce longitudinale (21a) augmente vers le côté d'extrémité avant,
une surface de vis-à-vis surélevée (21f), surélevée vers l'intérieur au-delà d'une surface faisant face au côté d'extrémité de base (21e) d'une extrémité de base de la pièce longitudinale (21a), est formée sur une surface dirigée vers l'intérieur de la pièce longitudinale (21a) où est positionnée la section effilée de prise (21d), et
dans un processus dans lequel la paire de pièces longitudinales (21a) est tirée dans l'extrémité distale (22a) du manchon (22) dans un état dans lequel les parties de bras (11, 12) de l'agrafe (1) sont positionnées entre la paire de pièces longitudinales (21a), la section effilée de prise (21d) vient en prise avec la bague de fixation (14) de l'agrafe (1) de façon à déplacer la bague de fixation (14) vers une extrémité de base de l'agrafe (1) .

2. Dispositif de retrait d'agrafe selon la revendication 1, dans lequel une surface effilée surélevée (21g) est formée dans une partie de limite entre la surface faisant face au côté d'extrémité de base (21e) et la surface de vis-à-vis surélevée (21f).

3. Dispositif de retrait d'agrafe selon la revendication 2, dans lequel la surface effilée surélevée (21g) est positionnée à une limite entre la section effilée de prise (21d) et l'extrémité de base de la pièce longitudinale (21a), ou positionnée plus près d'un côté d'extrémité de base que la limite le long de la pièce longitudinale (21a), l'extrémité de base de la pièce longitudinale (21a) étant la partie de la pièce longitudinale (21a) qui est positionnée plus près du côté d'extrémité de base que la section effilée de prise (21d).

4. Dispositif de retrait d'agrafe selon l'une quelconque des revendications 1 à 3, dans lequel une surface de vis-à-vis en cavité (221h), en cavité vers un côté opposé à la direction dans laquelle s'élève la surface de vis-à-vis surélevée (21f), est formée sur le côté d'extrémité avant, à l'avant de la surface de vis-à-vis surélevée (21f) le long de la pièce longitudinale (21a) .

5. Dispositif de retrait d'agrafe selon l'une quelconque des revendications 1 à 4, dans lequel la section effilée de prise (21d) est formée sur chacun des côtés dans la direction de la largeur à l'extrémité avant d'au moins l'une des pièces longitudinales (21a).
